(19) Europäisches Patentamt
European Patent Office
Office européen des brevets

(11) Veröffentlichungsnummer: **0 231 920**
**A2**

(12) # EUROPÄISCHE PATENTANMELDUNG

(21) Anmeldenummer: **87101379.3**

(22) Anmeldetag: **02.02.87**

(51) Int. Cl.⁴: **C07D 319/06**, C07D 321/06, C07D 317/26

(30) Priorität: **06.02.86 DE 3603662**

(43) Veröffentlichungstag der Anmeldung: **12.08.87 Patentblatt 87/33**

(84) Benannte Vertragsstaaten: **CH DE FR GB LI NL**

(71) Anmelder: **BASF Aktiengesellschaft**
**Carl-Bosch-Strasse 38**
**D-6700 Ludwigshafen(DE)**

(72) Erfinder: **Himmele, Walter, Dr.**
**Eichenweg 14**
**D-6909 Walldorf(DE)**
Erfinder: **Brenner, Karl**
**Riedsaumstrasse 40**
**D-6700 Ludwigshafen(DE)**
Erfinder: **Eggersdorfer, Manfred, Dr.**
**Hannongstrasse 18**
**D-6710 Frankenthal(DE)**
Erfinder: **Halbritter, Klaus, Dr.**
**Schwarzwaldstrasse 19**
**D-6800 Mannheim 1(DE)**

(54) **Acetalisierte oder ketalisierte Dihydroxyaldehyde, Verfahren zu ihrer Herstellung und diese enthaltende Lösungen.**

(57) Es wird vorgeschlagen ein Verfahren zur Herstellung acetalisierter oder ketalisierter Dihydroxyaldehyde der allgemeinen Formel I

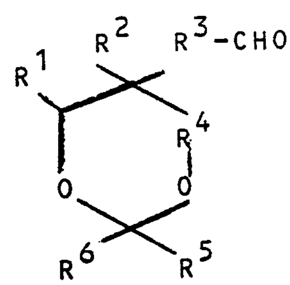

(I),

worin:
$R^1$, $R^2$, $R^5$ und $R^6$ gleich oder verschieden sind und jeweils für Wasserstoff, einen aliphatischen Rest, einen substituierten oder unsubstituierten aromatischen Rest, einen Aralkylrest oder Arylalkenylrest stehen,
oder worin $R^1$ und $R^2$ und/oder $R^5$ und $R^6$ jeweils gemeinsam auch für ein Ringsystem stehen können,
und $R^1$ auch einen Alkoxyrest bedeuten kann,
und worin $R^3$ und $R^4$ gleich oder verschieden sind und für eine Einfachbindung oder einen Alkylenrest stehen,
daß dadurch gekennzeichnet ist, daß man einen Alkohol der allgemeinen Formel II

EP 0 231 920 A2

Xerox Copy Centre

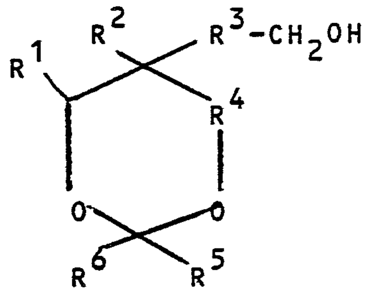

(II),

worin $R^1$, $R^2$, $R^3$, $R^4$, $R^5$ und $R^6$ die vorstehend genannten Bedeutung besitzen,
mit Sauerstoff oder einem Sauerstoff enthaltenden Gas in der Gasphase über einem Katalysator der Gruppe 1B des Periodensystems der Elemente bei Temperaturen zwischen 300 und 700°C und einer mittleren Verweilzeit von 0,0005 bis 1 Sekunden, umsetzt.

# Acetalisierte oder ketalisierte Dihydroxyaldehyde, Verfahren zu ihrer Herstellung und diese enthaltende Lösungen

Die Erfindung betrifft acetalisierte oder ketalisierte Dihydroxyaldehyde, ein Verfahren zu ihrer Herstellung und diese Verbindungen enthaltende Lösungen.

1,2-und 1,3-Dihydroxyaldehyde sind zentrale Zwischenprodukte zur Herstellung von Verbindungen mit 1,2-bzw. 1,3-Dihydroxy-Fragment, wozu biologisch interessante Vertreter der Pheromone, Makrolide, Fungizide und die Kohlenhydrate gehören.

Die 1,2-und 1,3-Dihydroxyaldehyde werden wegen ihrer Reaktivität und Instabilität in der Regel als acetalisierte oder ketalisierte Derivate hergestellt und in Synthesen eingesetzt.

Ein Beispiel für die schwierige Synthese eines nicht geschützten 1,2-Dihydroxyaldehyds stellt der Glycerinaldehyd dar: die Oxidation von Glycerin führt in schlechter Ausbeute zu einem Gemisch von Glycerinaldehyd und Dihydroxyaceton (Beilstein EI, 427).

Deshalb versuchte man, zwei Hydroxylfunktionen in Glycerin bzw. Trihydroxverbindungen als Acetal oder Ketal zu schützen und die verbleibende Hydroxymethylengruppe zur Aldehyd-Funktion zu oxidieren. Entsprechende Versuche, Acetale und Ketale des Glycerins bzw. der höheren Homologen zu oxidieren, wurden jedoch als nicht erfolgreich oder extrem aufwendig beschrieben (J. Chem. Eng. Data 9 (1964), Nr. 1, S. 99).

So ist aus Bull. Chem. Soc. Jap. 45 (1972) 8, 2620 bekannt, daß man Glycerinacetonid mit Phosgen zum Chlorkohlensäureester umsetzen und daraus nach Oxidation mit Dimethylsulfoxid und anschließender Behandlung mit Amberlit Glycerinaldehydacetonid gewinnen kann. Das Verfahren liefert beim einfachsten Baustein, dem Glycerinaldehydacetonid, eine Ausbeute von rund 33 %.

Nach JP-A-5 8203-983 kann die Ausbeute bei anderen Derivaten auf bis zu 85 % gesteigert werden, doch müssen dann extrem aufwendige Reaktionsbedingungen, wie Temperaturen von -78°C und Reagenzien, wie Oxalylchlorid, Verwendung finden.

Ein weiterer Zugang zu ketalisierten Dihydroxyaldehyden besteht in der oxidativen Spaltung von Zuckerketalen z.B. von Mannitol mit Bleitetraacetat (J. Biol. Chem. 128, 463, 1939). Dieser Weg liefert zwar für einzelne Verbindungen gute Resultate, ist aber auf Grund der Einsatzmaterialien wirtschaftlich aufwendig.

Der Erfindung liegt die Aufgabe zugrunde, neue acetalisierte oder ketalisierte Dihydroxyaldehyde, sowie ein Verfahren zu ihrer Herstellung zu schaffen, das die Herstellung dieser Verbindungen auf einfacherem Wege mit höherer Ausbeute und Reinheit erlaubt.

Diese Aufgabe wird gelöst durch die Lehre der Ansprüche.

Gegenstand der Erfindung ist somit ein Verfahren zur Herstellung acetalisierter oder ketalisierter Dihydroxyaldehyde der allgemeinen Formel I

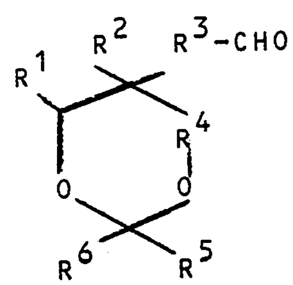 (I),

worin:
$R^1$, $R^2$, $R^5$ und $R^6$ gleich oder verschieden sind und jeweils für Wasserstoff, einen aliphatischen Rest, einen substituierten oder unsubstituierten aromatischen Rest, einen Aralkylrest oder Arylalkenylrest stehen,
oder worin $R^1$ und $R^2$ und/oder $R^5$ und $R^6$ jeweils gemeinsam auch für ein Ringsystem stehen können,
und $R^1$ auch einen Alkoxyrest bedeuten kann,
und worin $R^3$ und $R^4$ gleich oder verschieden sind und für eine Einfachbindung oder einen Alkylenrest stehen,

das dadurch gekennzeichnet ist, daß man einen Alkohol der allgemeinen Formel II

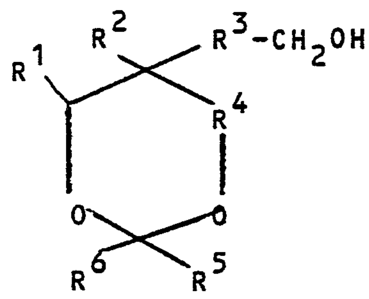

(II),

worin $R^1$, $R^2$, $R^3$, $R^4$, $R^5$ und $R^6$ die vorstehend genannten Bedeutungen besitzen, mit Sauerstoff oder einem Sauerstoff enthaltenden Gas in der Gasphase über einen Katalysator der Gruppe 1B des Periodensystems der Elemente bei Temperaturen zwischen 300 und 700°C und einer mittleren Verweilzeit von 0,0005 bis 1 Sekunden, umsetzt.

Gegenstand der Erfindung sind auch acetalisierte oder ketalisierte Dihydroxyaldehyde der allgemeinen Formel Ia:

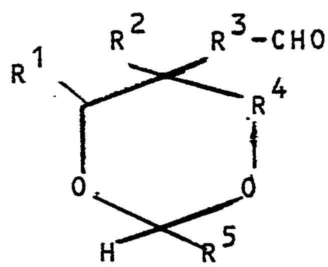

(Ia),

worin die Reste $R^1$, $R^2$, $R^3$, $R^4$, und $R^5$ gleich oder verschieden sind und jeweils für Wasserstoff, einen aliphatischen Rest, einen substituierten oder unsubstituierten aromatischen Rest, einen Aralkylrest oder Arylalkenylrest stehen,
oder worin $R^1$ und $R^2$ gemeinsam auch für ein Ringsystem stehen können, und $R^1$ auch einen Alkoxyrest bedeuten kann,
und worin $R^3$ und $R^4$ gleich oder verschieden sind und für eine Einfachbindung oder einen Alkylenrest stehen.

Im Vergleich zu den bekannten Verfahren liefert das erfindungsgemäße Verfahren überraschend auf einfacherem Wege ein besseres Ergebnis im Hinblick auf Ausbeute und Reinheit des Endstoffes.

In den Verbindungen der allgemeinen Formeln I, Ia und II können die Reste $R^1$, $R^2$, $R^5$ und $R^6$ für Wasserstoff, einen aliphatischen Rest, einen substituierten oder unsubstituierten aromatischen Rest, einen Aralkylrest oder einen Arylalkenylrest bestehen, oder aber die Reste $R^1$ und $R^2$ bzw. $R^5$ und $R^6$ können gemeinsam auch ein Ringsystem bilden. Der Rest $R^1$ kann darüber hinaus auch für einen Alkoxyrest stehen.

Als aliphatische Reste kommen Kohlenwasserstoffreste in Betracht, insbesondere geradkettige und verzweigte Kohlenwasserstoffreste mit 1 -30 Kohlenstoffatomen, insbesondere 1 -10 Kohlenstoffatome. Besonders bevorzugt sind Alkylreste mit 1 -6 Kohlenstoffatomen. Beispielhaft zu nennen sind Methyl-, Ethyl-, n-Propyl-, i-Propyl-, Butyl-, Pentyl-und Hexylreste. Diese Reste können auch cyclisch sein, sie können gewünschtenfalls auch substituiert sein, beispielsweise durch Halogen, Alkoxy, ins besondere $C_1$-$C_4$-Alkoxy. Die Rest können auch durch Heteroatome, beispielsweise durch Sauerstoff, Schwefel oder Stickstoffatome, unterbrochen sein. Als substituierte oder unsubstituierte aromatische Reste kommen insbesondere substituierte oder unsubstituierte Phenylreste in Betracht. Als Substituenten kommen insbesondere Halogen, Alkyl-(insbesondere $C_1$-$C_6$-Alkyl), oder Alkoxy-(insbesondere $C_1$-$C_6$-Alkoxy) Reste in Betracht. Bei den Aralkylresten kann es sich um solche handeln, welche einen Phenylrest aufweisen, wobei der Alkylrest beispielsweise 1 -6 Kohlenstoffatome aufweist. Bei den Arylalkenylresten kommen insbesondere solche in Betracht, welche einen substituierten oder unsubstituierten Phenylrest aufweisen. Bei diesen Resten kann der Alkenylrest beispielsweise 2 -10 Kohlenstoffatome aufweisen.

Wenn die Reste $R^1$ und $R^2$ bzw. $R^5$ und $R^6$ ein Ringsystem bilden, so kann es sich beispielsweise um 5- oder 6-Ringsysteme handeln, welche beispielsweise durch Heteroatome, z.B. Sauerstoff, Schwefel oder Stickstoff, unterbrochen sein können. Diese Ringsysteme können gesättigt oder ungesättigt sein.

Soweit der Rest $R^1$ auch für Alkoxy stehen kann, kommen hier insbesondere Alkoxyreste mit 1 -6 Kohlenstoffatomen in Betracht. Beispielhaft zu nennen sind Methoxy, Ethoxy, Propoxy, Butoxy, Pentoxy und Hexoxy.

In den Verbindungen der allgemeinen Formel I, Ia und II können die Reste $R^3$ und $R^4$ gleich oder verschieden sein und für eine Einfachbindung oder einen Alkylenrest stehen. Als Alkylenreste kommen insbesondere für den Rest $R^4$ ein $C_1$-$C_3$-Alkylenrest in Betracht. Der Rest $R^3$ kann insbesondere ein $C_1$-$C_{10}$-Alkylenrest sein. Wenn der Rest $R^4$ für eine Einfachbindung steht, weisen die Hydroxyaldehyde der allgemeinen Formeln I, Ia und II ein Fünfringgerüst auf.

Für die Umsetzung wird Alkohol der allgemeinen Formel II oder Alkohol der allgemeinen Formel II zusammen mit einem Lösemittel in Dampfform und vorteilhaft im Gemisch mit Inertgas dem Reaktionsraum zugeführt. Als unter Reaktionsbedingungen inerte Lösemittel werden zweckmäßig Tetrahydrofuran, Dioxan, Toluol oder $H_2O$ verwendet. Das Gewichtsverhältnis Lösemittel zu Alkohol beträgt vorteilhaft 0,5 bis 4 : 1. Als Inertgas können Edelgase, bevorzugt jedoch Stickstoff, Kohlenoxid und/oder Kohlendioxid verwendet werden. Das Molverhältnis von Inertgas zu Sauerstoff beträgt mindestens 4,4, zweckmäßig 4,4 bis 20 : 1, wobei sich die Angaben bezüglich Inertgas stets auf die Gesamtmenge, d.h. des Inertgasanteils der bevorzugt verwendeten Luft beziehen.

Als oxidierendes Agens lassen sich sowohl der reine Sauerstoff als auch Sauerstoff enthaltende Gase, insbesondere Luft, verwenden. Sauerstoff, in der Regel in Gestalt von Luft und Alkohol werden zweckmäßig im Molverhältnis von 0,2 bis 1,0, insbesondere 0,3 bis 0,7 Mol Sauerstoff je Mol Alkohol angewandt.

Die Verweilzeit des Gasgemisches im Reaktionsraum beträgt 0,0005 bis 1, bevorzugt 0,001 bis 0,05 Sekunden. Aufgrund der kurzen Verweilzeit werden Nebenreaktionen fast vollständig unterdrückt, und aufgrund der Reaktionstemperaturen wird das Ausgangsprodukt quantitativ umgesetzt.

Als Katalysator kann man einen solchen der Gruppe 1B des Periodensystems der Elemente einsetzen. Beispiele für geeignete Katalysatoren sind Silber, Kupfer und Gold, wobei auch Mischungen dieser Metalle verwendet werden können. Besonders bevorzugt ist ein Silberkatalysator. Wenn man eine Katalysatorschüttung verwendet, kann die Schichtdicke des Katalysators zweckmäßig 10 -40, vorzugsweise 15 -30 mm betragen. Die Katalysatorteilchen werden nach abnehmender Korngröße auf ein Netz aus Silber oder Edelstahl in einem vertikal aufgestellten Reaktor geschichtet. Als Katalysatorteilchen eignen sich Teilchen der Korngröße 0,1 bis 5 mm.

Die Standzeit des Katalysators mit konstanter Aktivität und Selektivität beträgt 3 Monate und mehr. Der Katalysator wird zweckmäßig mit 0,2 bis 2 t, insbesondere 0,3 bis 1,5 t Alkohol je $m^2$ Katalysatorquerschnitt und Stunde belastet. Die Raum-Zeit-Ausbeuten betragen 15 bis 60 kg des entsprechenden acetalisierten oder ketalisierten Dihydroxyaldehyds pro Liter Reaktionsvolumen und Stunde.

Für die oxidative Dehydrierung leitet man das Gasgemisch aus Alkohol, Luft und Inertgas in den genannten Mengen bei Temperaturen von 300 bis 700°C, vorzugsweise bei 520 bis 650°C, durch den Katalysator. Die Reaktion wird im allgemeinen bei Drücken zwischen 0,8 und 2 bar, vorzugsweise 1,05 und 1,5 bar, kontinuierlich durchgeführt.

Die aus dem Reaktor austretenden Reaktionsgase werden im Gleichstrom mit einem inerten Lösungsmittel oder dem Produkt-enthaltenden Lösungsmittelgemisch als Quenchflüssigkeit rasch auf Raumtemperatur abgekühlt. Als Lösungsmittel wählt man zweckmäßig ein mit dem acetalisierten oder ketalisierten Dihydroxyaldehyd mischbares, mit Wasser zwei-Phasen bildendes Lösungsmittel, z.B. aus der Verbindungsklasse der Kohlenwasserstoffe, substituierten Kohlenwasserstoffe oder Ether wie Hexan, Toluol oder Methyl-tert.-butylether. Das Gemisch wird zusammen mit den Reaktionsgasen über eine mit Glasringen gefüllte Säule zum vollständigen Lösen der Produkte im Lösungsmittel geführt.

Durch geeignete Wahl der Quenchflüssigkeit, der Temperatur und der Menge Quenchflüssigkeit können die kondensierbaren Anteile vollständig gewonnen werden.

Die so erhaltenen Lösungen der acetalisierten oder ketalisierten Dihydroxyaldehyde können unmittelbar für organische Synthesen eingesetzt werden, oder es können aus den Lösungen die reinen acetalisierten oder ketalisierten Dihydroxyaldehyde durch fraktionierende Destillation gewonnen werden. Die gereinigten Produkte werden zweckmäßigerweise wegen der Möglichkeit der Trimerisierung zügig weiterverarbeitet.

Die Erfindung wird durch die nachstehenden Beispiel weiter erläutert.

Beispiel 1

Herstellung von Glycerinaldehyd-acetaldehydacetal

Man verwendet eine Anlage mit Zuführungen für Luft und Inerte, sowie für Alkohol auf den Kopf eines senkrecht stehenden Rohrreaktors. Der Rohrreaktor enthält eine Silberkristall-Katalysatorschicht der folgenden Zusammensetzung:

| | Anteil am Katalysator Gew.-% | Korngröße mm |
|---|---|---|
| Schicht 1 | 20 | 0,2 - 0,4 |
| Schicht 2 | 50 | 0,4 - 0,75 |
| Schicht 3 | 30 | 0,75 - 7,0 |

Der Reaktor geht über in den Quenchraum, der den Kopf einer Füllkörperkolonne bildet. Daran schließt sich ein Phasentrenngefäß an. Die organische Phase wird als Quenchflüssigkeit mit einer Pumpe über einen Wärmeaustauscher und eine Düse unmittelbar hinter der Katalysatorschicht in die heißen Gase gesprüht, die im Quenchraum auf Raumtemperatur abgekühlt und kondensiert bzw. gelöst werden.

Dem Reaktor werden pro Stunde ein Gemisch von 180 g Glycerin-acetaldehydacetal und 133 l Luft mit einer Eintrittstemperatur von 270°C zugeführt. Die Reaktionstemperatur beträgt 580°C, die durchschnittliche Verweilzeit im Katalysatorbett liegt bei 0,02 Sekunden.

Die organische Phase wird anschließend von Spuren Säure freigewaschen und getrocknet. Sie kann entweder direkt für Synthesen eingesetzt werden oder wird zur Reinisolierung der Glycerinaldehydacetaldehydacetals fraktionierend destilliert. Der Umsatz ist vollständig, die Ausbeute beträgt 72 % d. Th. $Kp._{60}$78-80°C.

Beispiel 2

Herstellung von 3,4-Dihydroxy-butyraldehyd-acetaldehyd-acetal

Analog Beispiel 1 werden pro Stunde 160 g 1,3,4-Trihydroxy-butanol-acetaldehyd-acetal und 120 l Luft bei einer Reaktionstemperatur von 590°C über den Silberkristall-Katalysator gefahren. Aufarbeitung des Rohaustrags durch fraktionierende Destillation liefert eine Ausbeute von 68 % d. Th. $Kp_{70}$140°C.

Beispiel 3

Herstellung von (1,1-Bishydroxymethyl)-butyraldehyd-isobutyraldehyd-aceta l

Analog Beispiel 1 werden dem Reaktor pro Stunde ein Gemisch von 150 g Trimethyolpropan-acetaldehydracetal und 110 l Luft mit einer Eintrittstemperatur von 270°C zugeführt.

Die Reaktionstemperatur beträgt 680°C. Aufarbeitung analog Beispiel 1. Ausbeute 50 % d. Th. $Kp_{50}$130-132°C.

Beispiel 4

Herstellung von 2-Methyl-glycerinaldehyd-acetaldehyd-acetal

Analog Beispiel 1 wird in den Reaktor eine Lösung von 50 Gew.-% 2-Methylglycerin-acetaldehyd in THF (250 g/h) und 100 l Luft pro Stunde geblasen. Die Reaktionstemperatur liegt bei 620°C. Der Reaktionsaustrag wird getrocknet und destillativ aufgearbeitet. Ausbeute 75 % d. Th.. $Kp_{30}$62°C.

Beispiel 5

Herstellung von Glycerinaldehyd-acetonid

Analog Beispiel 1 werden pro Strunde 180 g Glycerin-aceton-ketal und 140 l Luft über einen Katalysator aus Kupferkristallen der Korngröße 0,2 bis 0,75 mm gefahren. Die Reaktion verläuft bei einer Temperatur von 520°C. Die Aufarbeitung des Reaktionsaustrags erfolgt analog Beispiel 1. Die Ausbeute nach Destillation beträgt 65 % der Theorie. $Kp_{34}$60°C.

## Ansprüche

1. Verfahren zur Herstellung acetalisierter oder ketalisierter Dihydroxyaldehyde der allgemeinen Formel I

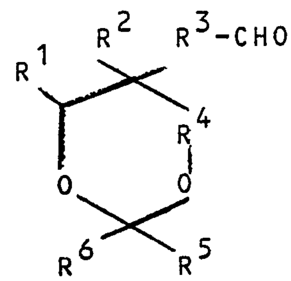

(I),

worin:
$R^1$, $R^2$, $R^5$ und $R^6$ gleich oder verschieden sind und jeweils für Wasserstoff, einen aliphatischen Rest, einen substituierten oder unsubstituierten aromatischen Rest, einen Aralkylrest oder Arylalkenylrest stehen, oder worin $R^1$ und $R^2$ und/oder $R^5$ und $R^6$ jeweils gemeinsam auch für ein Ringsystem stehen können, und $R^1$ auch einen Alkoxyrest bedeuten kann, und worin $R^3$ und $R^4$ gleich oder verschieden sind und für eine Einfachbindung oder einen Alkylenrest stehen, dadurch gekennzeichnet, daß man einen Alkohol der allgemeinen Formel II

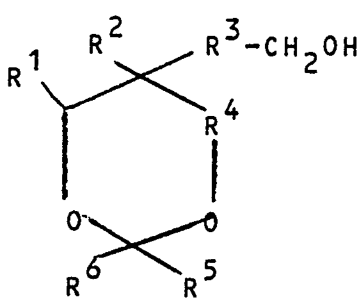

(II),

worin $R^1$, $R^2$, $R^3$, $R^4$, $R^5$ und $R^6$ die vorstehend genannten Bedeutungen besitzen, mit Sauerstoff oder einem Sauerstoff enthaltenden Gas in der Gasphase über einen Katalysator der Gruppe 1B des Periodensystems der Elemente bei Temperaturen zwischen 300 und 700°C und einer mittleren Verweilzeit von 0,0005 bis 1 Sekunden, umsetzt.

2. Verfahren nach Anspruch 1, dadurch gekennzeichnet, daß man die Reaktionsgase schnell abkühlt.

3. Verfahren nach einem der Ansprüche 1 oder 2, dadurch gekennzeichnet, daß man den Alkohol der allgemeinen Formel II zusammen mit einem Lösemittel in Dampfform in die Reaktion einbringt.

4. Verfahren nach einem der Ansprüche 1 bis 3, dadurch gekennzeichnet, daß man in Gegenwart eines Inertgases umsetzt.

5. Verfahren nach Anspruch 4, dadurch gekennzeichnet , daß man ein Molverhältnis von Inertgas zu Sauerstoff von mindestens 4,4 : 1 wählt.

6. Verfahren nach einem der Ansprüche 1 bis 5, dadurch gekennzeichnet, daß man als Sauerstoff enthaltendes Gas Luft verwendet und ein Molverhältnis von Sauerstoff zu Alkohol der allgemeinen Formel II von 0,2 bis 1,0 : 1 wählt.

7. Verfahren nach einem der Ansprüche 1 bis 6, dadurch gekennzeichnet, daß man als Katalysator kristallines, metallisches Silber einsetzt.

8. Verfahren nach einem der Ansprüche 1 bis 7, dadurch gekennzeichnet , daß man bei einem Druck zwischen O,8 und 2 bar arbeitet.

9. Verfahren nach einem der Ansprüche 2 bis 8, dadurch gekennzeichnet, daß man zur Abkühlung der Reaktionsgase diese mit einem Lösemittel behandelt und darin löst.

1O. Acetalisierte oder ketalisierte Dihydroxyaldehyde der allgemeinen Formel Ia:

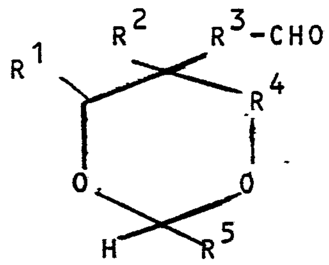

(Ia),

worin die Reste $R^1$, $R^2$, $R^3$, $R^4$ und $R^5$ die in Anspruch 1 angegebenen Bedeutungen besitzen.

11. Lösung von acetalisierten oder ketalisierten Dihydroxyaldehyden der allgemeinen Formeln I gemäß Anspruch 1 oder Ia gemäß Anspruch 1O in einem inerten Lösungsmittel.

12. Lösung nach Anspruch 11, erhältlich nach dem Verfahren gemäß Anspruch 9.